# EUROPEAN PATENT APPLICATION

(11) **EP 2 130 617 A1**
(43) Date of publication of application: **09.12.2009**
(21) Application number: 08165558.1
(22) Date of filing: 30.09.2008
(51) Int. Cl.: B09B 1/00, B09B 3/00

(54) **Method for intensification of methane production in refuse collection depot**

(30) Priority: 02.06.2008 PL 38533708
(71) Applicant: Politechnika Lubelska, 20-219 Lublin (PL)
(72) Inventor: Pawlowska, Malgorzata, 20-027 Lublin (PL); Siepak, Jerzy, 60-687 Poznan (PL); Pawlowski, Lucjan, 20-027 Lublin (PL); Pleczynski, Józef, 61-606 Poznan (PL)
(74) Representative: Kaminski, Piotr

(57) **Abstract**

Method for intensification of methane production in refuse collection depot is **characterised in that** sodium acetate in the amount of 0,5-3 kg CH₃COONa/m² is sprinkled over the depot, preferably in concentration of 10% and in amount of 1 kg CH₃COONa/m².

## Description

The present invention relates to a method for intensification of methane production in refuse collection depot. Refuse collection depot is the bioreactor, where bio-degradable fraction is microbiologically broken-down to simple inorganic and organic compounds. During this process also processes of chemical and physical nature occur.

Decomposition of bio-fraction in the refuse collection depot lasts tens of years and consists of several phases. Most generally one can distinguish two main phases: aerobic and anaerobic. In the anaerobic phase one can distinguish the phase of acidic fermentation and the stable and unstable methane fermentation. Some authors: Bozkurt S., Moreno L., Neretnieks I. in Long-term Processes in Waste Deposits, The Science of the Total Environment, 250, 101-121, 2000, additionally distinguish the humus phase, which is defined as a maturation phase (Vaidya R.D., Solid Waste Degradation, Compaction and Water Holding Capacity, Virginia Polytechnic Institute and State University, 2002). It begins once the methane production in the depot is finished. The humus phase is not well described in the literature and the processes occurring during this phase have not been thoroughly studied. The composition of biogas generated in the particular phases differ significantly.

During the first phase of waste degradation - aerobic - (Phase 1), which lasts several days, the oxygen is accessible to microorganisms. In this phase hydrolysis of complex organic compounds, such as hydrocarbons, proteins, fats occurs and as a result monosaccharides, glycerol, higher fatty acids and amino acids are produced. Hydrolysis is the first stage of decomposition of organic matter. The products of this process are soluble and are easily assimilated by microorganisms. Hydrolysis is carried out by saprophytic bacteria, which produce extracellular enzymes with which insoluble compounds are treated. As a result of these reactions soluble monomers are created. Hydrolysis usually lasts long, which is why when the contents of organic substances is substantial, the methanogenesis can be limited.

When the oxygen contents drops, the phase of anaerobic decomposition begins (Phase II). During this stage the dominant microorganisms are those, which are facultative and obligatory anaerobes (Bozkurt S., Moreno L., Neretnieks I. in Long-term Processes in Waste Deposits, The Science of the Total Environment, 250, 101-121, 2000). The first stage of anaerobic phase is called acidogenic phase, i.e. acidic, because during this phase the products of hydrolysis are processed by acidogenic bacteria into organic acids: formic acid, acetic acid, propionic and butyric acids. In addition alcohols are formed: methanol, ethanol, but also hydrogen, carbon dioxide, ammonia and hydrogen sulphide.

In subsequent phase - acetogenic - acetic acid is formed and it is the main substrate, from which methane will be created later on. Decomposition of fatty acids, organic acids and alcohols causes release of hydrogen. Bacteria from group of *Syntrophobacter* and *Syntrophomonas* are considered as acetogenic bacteria, they are characterised by long generation time and capability of growth on solid media. In the course of fermentation the mentioned bacteria utilise for growth the compounds produced during the acidic fermentation and convert these products into acetates, carbon dioxide and hydrogen. Bacterial growth in this phase can be inhibited by H₂. Hydrogen present in this environment dislodges acetogenic bacteria which can lead to accumulation of volatile organic acids, lowering reaction and in consequence it can stop the growth of methanogenic bacteria (Janosz-Rajczyk M. *et al* "Selected elementary processes in environmental engineering" Publication of Politechnika Cz 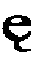 stochowska 2004, ISBN 83-7193-271-5).

Transition into the phase of anaerobic decomposition (Phase III) is linked with appearance of methane in biogas. Initially the methane content is variable, with the time though it stabilises to amount around 45-60% volumetric. The phase of stable methane production can last between 8 and 40 years. However, biogas utilisation for energy purposes is economically viable in the period between 5 and 20 years. After that the methane contents drops gradually until it disappears completely.

Methane fermentation aims at conversion of organic compounds at different oxidation levels into gaseous end products, mainly methane and carbon dioxide and the intermediate products such as alcohols and fatty acids. Intermediate products, both mineral as well as organic serve as source of carbon and energy for microorganisms. During the methanogenesis also occur: the aerobic ammonification, reduction of nitrates as well as the microbiological reduction of sulphates into hydrogen sulphide.

Biogas can be used for energy purposes when the methane content exceeds 30%. Release of biogas with lower methane content to atmosphere will cause adverse environmental effect. The influence of methane on greenhouse effect is 20 times stronger that that of carbon dioxide, commonly considered as a greenhouse gas.

Attempts are made to limit methane emission by its oxidation in the overlay of the depot or in specially designed biofilters. These methods allow for limiting the effect of methane emission on greenhouse effect, but the energy in form of methane contained in the biogas is irretrievably lost, which in view of deficits in the sources of fossil fuel is a great waste.

From point of view of economy it is desirable, that the biogas containing more that 30 % of methane be produced for as long as possible. For this reason attempts are made to intensify the methane emission from refuse collection depots.

US patent 6471443 describes a method which involves forcing of overheated steam into the waste depot through a well hollowed in it. According to this patent, steam increases the methane output and hence allows for its better utilisation. The drawback of this method is high energy consumption necessary for generation of steam.

US patent 5695641 describes different method for intensification of methane production. According to this patent gaseous NH₃ or its aqueous solution is forced into the refuse in the depot. The drawback of this method is the difficulty in adjusting the right amount of ammonia to avoid overdosage.

Other method was described in US patent 4320802. According to this patent biogas of lower calorific value is forced into depleted well in order to force out the crude oil.

The essence of the present method for intensification of methane production in refuse collection depot is that one sprinkles over the depot the sodium acetate in the amount of 0,5-3 kg CH₃COONa/m², preferably in concentration of 10% and in amount of 1 kg CH₃COONa/m².

The beneficial effect of the method according to present invention is that one can increase the methane content in biogas to over 30%.

### Example:

On the separated quarter of refuse collection depot, having dimensions of 8x30 m=240 m² and thickness between 7 and 9 m, 10% solution of sodium acetate was sprinkled in the amount of 2640 litters per the area as mentioned. Before sodium acetate treatment the methane contents in the emitted biogas was in range between 13 and 17%. After the sodium acetate application, the methane concentration started to rise and amounted as follows: 20% as measured after 17 days, 39,9% as measured after 90 days. After 210 the methane concentration stabilised at the level of 50±3%.

## Claims

1. Method for intensification of methane production in refuse collection depot **characterised in that** sodium acetate in the amount of 0,5-3 kg CH₃COONa/m² is sprinkled over the depot, preferably in concentration of 10% and in amount of 1 kg CH₃COONa/m².
